(19) Europäisches Patentamt
European Patent Office
Office européen des brevets

(11) Veröffentlichungsnummer : **0 105 525**
**B1**

(12) # EUROPÄISCHE PATENTSCHRIFT

(45) Veröffentlichungstag der Patentschrift : **10.09.86**

(21) Anmeldenummer : **83109919.7**

(22) Anmeldetag : **04.10.83**

(51) Int. Cl.[4] : **C 07 C161/00**, C 07 D295/20, C 07 C149/247, C 07 K 1/06, C 07 K 7/06

(54) **Neue Thiohydrazin-1,2-dicarbonsäure-Derivate, Verfahren zu ihrer Herstellung und ihre Verwendung.**

(30) Priorität : **05.10.82 DE 3236849**

(43) Veröffentlichungstag der Anmeldung : **18.04.84 Patentblatt 84/16**

(45) Bekanntmachung des Hinweises auf die Patenterteilung : **10.09.86 Patentblatt 86/37**

(84) Benannte Vertragsstaaten : **AT BE CH DE FR GB LI**

(56) Entgegenhaltungen :
**FR-A- 2 177 001**
**TETRAHEDRON LETTERS, Nr. 56, Dezember 1968, Seiten 5907-5908, Pergamon Press, Oxford, GB T. MUKAIYAMA u.a.: "A convenient method for the preparation of unsymmetrical disulfides by the use of diethyl azodicarboxylate"**
**CHEMICAL ABSTRACTS, Band 97, Nr. 11, 13. September 1982, Seite 817, Nr. 92734w, Columbus, Ohio, US E. WUENSCH u.a.: "A new method for the selective synthesis of unsymmetrical cystine peptides"**
**CHEMICAL ABSTRACTS, Band 98, Nr. 9, 28. Februar 1983, Seite 297, Nr. 68154g, Columbus, Ohio, US E. WUENSCH u.a.: "1-(tert-Butylthiol)-1,2-hydrazine dicarboxylic acid derivatives. New reagents for the introduction of the S-tert-butylthio group into cysteine and cysteine derivatives**

(73) Patentinhaber : **Max-Planck-Gesellschaft zur Förderung der Wissenschaften e.v.**
**Bunsenstrasse 10**
**D-3400 Göttingen (DE)**

(72) Erfinder : **Wünsch, Erich, Prof. Dr.**
**Midgardstrasse 16**
**D-8132 Tutzing (DE)**

(74) Vertreter : **Lederer, Franz, Dr. et al**
**Patentanwälte Dr. Franz Lederer Dipl.-Ing. Reiner F. Meyer-Roxlau Lucile-Grahn-Strasse 22**
**D-8000 München 80 (DE)**

**EP 0 105 525 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Es sind einige Verfahren bekannt, um die Mercapto-Gruppe des Cysteins reversibel zu maskieren. So kann man das Cystein in das S-Benzyl-Derivat umwandeln, doch müssen zur Wiederabspaltung dieser Schutzgruppe Bedingungen angewendet werden, die zu unerwünschten Sekundärreaktionen führen können. Die Maskierung der SH-Gruppe als S-Diphenylmethyl- oder S-Trityl-Derivat engt andererseits die Verwendung zahlreicher anderer Schutzgruppen wegen der allzu leichten Spaltbarkeit dieser Thioäther stark ein. Auch die Verwendung von S-Äthylmercaptocystein, d. h. eines unsymmetrischen Disulfids mit einem Cystein-Rest (Bull. Chem. Soc. Japan 40, 2913 (1967)) für Peptidsynthesen verhindert nicht die bekannte Disproportionierung unsymmetrischer Disulfide in die symmetrischen. Auch kann hierbei eine Oxidation der Disulfidbindung unter Bildung von Sulfinsäurethioestern erfolgen.

Die Anmelderin hat daher bereits vor längerer Zeit (DE-PS 19 23 480) ein Verfahren erarbeitet, wobei die Mercapto-Gruppe des Cysteins durch Einführung der tert.-Butylmercapto-Gruppe geschützt wird. Bei diesem Verfahren wird zur Einführung der Schutzgruppe die Disulfid-Brücke des Cystins durch Zugabe von überschüssigem tert.-Butylmercaptan gespalten. Bei Raumtemperatur benötigt diese Reaktion wenigstens einige Tage bis zu etwa einer Woche. Obwohl sich diese Schutzgruppe äußerst bewährt hat, sind die bisher bekannten Methoden zur Einführung dieser Schutzgruppe problematisch, einerseits wegen der Dauer der vorstehend genannten Reaktion, andererseits wegen des im Überschuß anzuwendenden Mercaptans, welches, vor allem im großtechnischen Maßstab, bei Aufarbeitung des Überschusses infolge der argen Geruchsbelästigung ein großes Umweltproblem darstellt.

Aufgabe der Erfindung ist daher die Bereitstellung neuartiger Mittel und Methoden, um die tert.-Butylmercapto-Schutzgruppe bzw. ähnliche sperrige tert.-Alkylthio-Schutzgruppen in Thiole, insbesondere in Cystein, Cystein-Derivate und Cystein-Peptide, einzuführen.

Es wurde gefunden, daß 1-tert.-Alkyl-thiohydrazin-1,2-dicarbonsäure-Derivate der allgemeinen Formel I

$$\begin{array}{ccccccc} R^1 - S - & N & - CO - R^2 \\ & | & \\ H - & N & - CO - R^2 \end{array} \qquad (I)$$

in welcher

$R^1$ eine tertiäre Alkylgruppe, nämlich tert.-Butyl oder tert.-Amyl und

$R^2$ einen Ester- oder Amidrest bedeuten, leicht herstellbar sind und ein ausgezeichnetes Reagenz zur Einführung von tert.-Alkylthio-Schutzgruppen, insbesondere der tert.-Butyl- und der tert.-Amylthio-Gruppe darstellen.

Es ist seit langem bekannt (Teruaki Mukaiyama und Katsuji Takahashi : Tetrahedron Letters 56, 5907-5908, 1968) Diäthylazodicarboxylat mit n-Äthyl- und n-Amylmercaptan zur Herstellung von unsymmetrischen Disulfiden nach folgendem Reaktionsschema unter milden und neutralen Bedingungen umzusetzen :

$$RSH + C_2H_5O_2CN = NCO_2C_2H_5 \rightarrow C_2H_5O_2CNH{-}N(SR)CO_2C_2H_5 \qquad (I)$$

$$+ R'SH \rightarrow RSSR' + C_2H_5O_2CNH{-}NHCO_2C_2H_5 \qquad (I)$$

Mit sterisch gehinderten Alkylmercaptanen, beispielsweise mit tert.-Butylmercaptan, läuft diese bekannte Reaktion jedoch nicht.

Es wurde nun jedoch gefunden, daß auch mit tert.-Butylmercaptan eine quantitative Umsetzung erreicht werden kann, wenn man dem Reaktionsmilieu katalytische Mengen einer sehr starken Base zusetzt, wobei 1-tert.-Alkyl-thiohydrazin-1,2-dicarbonsäure-Derivate erhalten werden, die ein ausgezeichnetes Reagenz zur Übertragung der 1-tert.-Alkylthiogruppe darstellen.

In den erfindungsgemäßen 1-tert.-Alkyl-thiohydrazin-1,2-dicarbonsäure-Derivaten der allgemeinen Formel I stellt der Substituent $R^1$ eine für die Aufgabenstellung geeignete tertiäre Alkylgruppe dar, insbesondere die tert.-Butylgruppe, obwohl auch die tert.-Amylgruppe infrage kommen kann.

Der Substituent $R^2$ stellt einen geeigneten Ester- oder Amidrest dar. Geeignete Esterreste können von Alkylalkoholen, beispielsweise solchen mit 1-6 C-Atomen, insbesondere 1-4 C-Atomen, wie Methyl-, Äthyl-, Propyl-, n-Butyl-, tert.-Butylalkohol oder von Arylalkoholen wie Phenol oder von Aralkylalkoholen abstammen.

Geeignete Amidreste leiten sich von Ammoniak, Mono- oder Dialkylaminen, Mono- oder Diarylaminen oder cyclischen Aminen wie beispielsweise Morpholin ab, wobei die bevorzugten Alkylamine 1-6 C-Atome aufweisen und Phenyl das bevorzugte Aryl ist. Durch geeignete Auswahl der Ester- und Amidreste können die Löslichkeitseigenschaften der erfindungsgemäßen Reagenzien in Abhängigkeit von dem zu wählenden Reaktionsmilieu beeinflußt werden. So wurde gefunden, daß sich das Dimorpholid besonders zur Übertragung der tert.-Butylthiogruppe in wässrigem Medium eignet, wie auch in organischwässrigem Milieu wie Dioxan/Wasser, Acetonitril/Ammonacetatpuffer oder zweiphasig Essigester/Wasser.

Unter den Ester-Derivaten, die in organischen Lösungsmitteln gut löslich sind, wird der Di-tert.-Butylester besonders bevorzugt.

Die erfindungsgemäßen Reagenzien können hergestellt werden indem man ein tert.-Alkylmercaptan der allgemeinen Formel II

$$R^1—S—H \qquad (II)$$

in welcher $R^1$ der in Anspruch 1 angegebene Bedeutung besitzt, mit einem Azodicarbonsäure-Derivat der allgemeinen Formel III

$$R^2—OC—N = N—CO—R^2 \qquad (III)$$

in welcher $R^2$ die in Anspruch 1 angegebene Bedeutung besitzt, in einem inerten, aprotischen Lösungsmittel in Gegenwart einer katalytischen Menge einer sehr starken Base zur Umsetzung bringt.

Als inerte aprotische Lösungsmittel eignen sich beispielsweise Diäthyläther, Dioxan oder Tetrahydrofuran. Zahlreiche andere geeignete Lösungsmittel sind dem Fachmann bekannt.

Als sehr starke Basen, deren Anwesenheit in katalytischen Mengen erforderlich ist, kommen Alkali- oder Erdalkalialkoholate, -amide, -hydride oder -hydroxide in Frage. Als Beispiele können genannt werden Natrium- oder Kaliummethylat oder -äthylat, Natrium- oder Kaliumamid, Natrium- oder Kaliumhydrid oder Natrium- oder Kaliumhydroxid.

Die Reaktion wird zweckmäßigerweise zwischen Raumtemperatur und der Siedetemperatur des Lösungsmittels durchgeführt ; sie verläuft exotherm.

Die erfindungsgemäßen Reagenzien können nach Entfernung des Lösungsmittels durch Aus- und Umkristallisation isoliert werden. Sie sind gut lagerstabil.

Die erfindungsgemäßen Reagenzien eignen sich vorzüglich zur Übertragung des tert.-Butylthio-Restes an Thiole, z. B. Cystein, Cystein-Derivate und -Peptide. Mittels dieser Methode ist die Zugänglichkeit von S-tert.-Butylthiocystein erstmals nach einem sehr umweltfreundlichen (ohne Geruchsbelastung) Verfahren in hohen Ausbeuten möglich. S-tert.-Butylthiocystein wurde in der Zwischenzeit als äußerst günstiges Cystein-Derivat zur Synthese von Cystein- und Cystinpeptiden erkannt (Moroder, L., Gemeiner, M., Göhring, W., Jaeger, E., Thamm, P., und Wünsch, E. (1981) Biopolymers, 20, 17-37).

Zudem ermöglichen die erfindungsgemäßen 1-tert.-Butylthiohydrazin-1,2-dicarbonsäure-Derivate eine reversible Markierung der Cystein-Thiolfunktionen in Peptiden und Proteinen wie dies erforderlich ist

1) in der Aminosäure-Sequenz-Analyse,

2) in den chemischen oder enzymatischen Semisynthesen,

3) in der Synthese von Cystin- oder Cysteinpeptiden durch nachträglichen intermediären Schutz der Cystein-Thiol-funktion,

4) in gezielten Synthesen asymmetrischer Cystin-Peptide zum Abfangen überschüssiger Cystein-Komponente, wobei die erhaltene isolierbare S-geschützte Cystein-Komponente nach Abspalten der S-Schutzgruppe wieder verwendbar wird (Recycling Process).

Die gezielte Synthese von asymmetrischen Cystinpeptiden mittels der Sulfenohydrazid-Methode ist in der Literatur beschrieben (Wünsch, E., und Romani, S. (1982) Hoppe-Seyler's Z. Physiol. Chem. *363*, 449-453 ; Romani, S., Göhring. W., Moroder, L. und Wünsch, E., Proceedings of the 4th FRG-USSR Symposium on Chemistry of Peptides and Proteins, Tübingen, June 8-12, 1982 ; Wünsch, E., Romani, S. und Moroder, L., in Proceedings of the 17th European Peptide Symposium, Prag, August 29 — September 3, 1982). Die Erfindung umfaßt demnach auch solche Verfahren, in welchen die erfindungsgemäßen Reagenzien eingesetzt werden.

Um bei der asymmetrischen Cystinpeptidsynthese die zeitabhängige, thiolinduzierte Disproportionierung des asymmetrischen Cystinpeptids zu den symmetrischen Disulfiden zu unterdrücken, empfiehlt es sich entweder einen großen Überschuß an der aktivierten Sulfenohydrazidkomponente zu verwenden, oder die nicht umgesetzte Thiolkomponente aus dem Reaktionsgemisch heraus abzufangen, sobald die maximale Konzentration an asymmetrischem Cystinpeptid erreicht ist.

Die bisher als Thiolreagenzien verwendeten Maleimidderivate bilden jedoch irreversible Konjugate mit der SH-Komponente, was besonders bei größeren Peptiden einen nicht unbedeutenden Verlust darstellt. Verwendet man zum Blockieren der überschüssigen Thiolkomponente jedoch die erfindungsgemäßen Reagenzien, so kann man das tert.-Butylthio-geschützte Cysteinpeptid nahezu quantitativ zurückgewinnen.

## Beispiel 1

### Herstellung von 1-tert.-Butylthiohydrazin-1,2-dicarbonsäure-dimorpholid

a) Azodicarbonsäure-dimorpholid

Zur Lösung von 17,4 g (0,1 M) Azodicarbonsäurediäthylester in 100 ml Äther werden 17,4 g (0.2 M)

Morpholin in 100 ml Ather getropft. Der dabei ausgefallene orangefarbene Niederschlag wird 5 Stunden bei Raumtemperatur nachgerührt und abfiltriert. Aus dem Filtrat erhält man nach Einengen im Vakuum und Versetzen mit wenig Petroläther eine weitere Fraktion.

Ausbeute : 18,6 g (73 % d. Th.) ; Schmp. 141-143 °C
$C_{10}H_{14}N_4O_4$ (256,265)
Ber. : C 46,87 H 6,29 N 21,86
Gef. : C 46,49 H 6,30 N 21,67

b) Zu einer Lösung von 2,3 ml (0,02 M) tert.-Butylmercaptan und 5,12 g (0,02 M) Azodicarbonsäure-dimorpholid in 150 ml Tetrahydrofuran werden katalytische Mengen an Natriumethylat hinzugefügt ; die Reaktion verläuft exotherm. Man engt die Reaktionslösung i. Vak. auf die Hälfte ein und das Sulfenohydrazid (1-tert.-Butylthiohydrazin-1,2-dicarbonsäure-dimorpholid) kristallisiert nach Zugabe von Petroläther aus.

Ausbeute : 6,44 g (93 % d. Th.) ; Schmp. 152-154 °C
$C_{14}H_{26}N_4O_4S$ (346,448)
Ber. : C 48,54 H 7,56 N 16,17 S 9,25
Gef. : C 48,11 H 7,42 N 16,31 S 9,28

## Beispiel 2

Herstellung von 1-tert.-Butylthiohydrazin-1,2-dicarbonsäure-di-tert.-butylester

Zu einer Lösung von 1,15 g (0,005 M) Azodicarbonsäure-di-tert.-butylester und 0.57 ml (0.005 M) tert.-Butylmercaptan in 20 ml Äther fügt man eine katalytische Menge an Natriumethylat hinzu ; die Reaktion verläuft exotherm. Das Lösungsmittel wird i. Vak. entfernt und der ölige Rückstand aus n-Hexan umkristallisiert.

Ausbeute : 1,54 g (96 % d. Th.) ; Schmp. 92-96 °C
$C_{14}H_{28}N_2O_4S$ (320,45)
Ber. : C 52,47 H 8,81 N 8,74 S 10,00
Gef. : C 52,45 H 8,80 N 8,78 S 9,93

## Beispiel 3 (Anwendungsbeispiel)

S-tert.-Butylthio-cystein

Zu einer Suspension von 3,4 g (0.01 M) 1-tert.-Butylthiohydrazin-1,2-dicarbonsäure-dimorpholid in 40 ml argongesättigtem Wasser wird eine Lösung von 0,6 g (0.005 M) Cystein in 20 ml argongesättigten Wasser langsam zugetropft. Dabei entsteht eine klare Lösung, aus der sich nach einer Stunde S-tert.-Butylthio-cystein auszuscheiden beginnt. Nach 12 h filtriert man den Niederschlag ab und extrahiert aus dem Filtrat das Überschußreagenz mit Essigester. Die wässrige Phase wird i. Vak. eingeengt und beim Stehen in der Kälte kristallisiert eine weitere Fraktion aus.

Ausbeute : 84 % (d. Th.) ; Zers. ab 176 °C ;
$[\alpha]_D^{20°}$ : — 89,6° bzw. $[\alpha]_{546}^{20°}$ : — 106,7 (c = 1 in 1N HCl).
$C_7H_{15}NO_2S_2 \cdot 1/2\ H_2O$ (218,344)
Ber. : C 38,51 H 7,39 N 6,42 S 29,37
Gef. : C 38,35 H 7,36 N 6,83 S 29,17

## Beispiel 4 (Anwendungsbeispiel)

N-tert.-Butyloxycarbonyl-O-tert.-butyl-L-threonyl-L-alanyl-S-(tert.-butylthio)-L-cysteinyl-glycyl-L-glutaminyl-$N^{\varepsilon}$-tert.-butyloxycarbonyl-L-lysyl-O-tert.-butyl-L-seryl-L-prolin-tert. -butylester

Zu einer Lösung von 250 mg (0.78 mM) 1-tert.-Butylthiohydrazin-1,2-dicarbonsäure-di-tert.-butylester in 5 ml argongesättigtem Dimethylformamid wird unter Argonatmosphäre eine Lösung von 302 mg (0,26 mM) Boc-Thr ($Bu^t$)-Ala-Cys-Gly-Gln-Lys(Boc)-Ser($Bu^t$)-Pro-$OBu^t$ in 10 ml argongesättigtem Dimethylformamid getropft. Nach 12 h Rühren bei Raumtemperatur wird die Reaktionslösung i. Vak. eingeengt und mit Petroläther versetzt. Der Niederschlag wird abfiltriert und aus Methanol/Petroläther umkristallisiert.

Ausbeute : 300 mg (93 % d. Th.) ; Schmp. 192-194 °C ;
$[\alpha]_D^{20°}$ : — 56,8° bzw. $[\alpha]_{546}^{20°}$ : — 67,5° (c = 1 in Äthanol).
$C_{57}H_{102}N_{10}O_{16}S_2 \cdot H_2O$ (1 265,66)

Ber.: C 54,09 H 8,29 N 11,07 S 5,06
Gef.: C 54,17 H 8,34 N 10,69 S 4,82

## Patentansprüche

1. 1-tert.-Alkyl-thiohydrazin-1,2-dicarbonsäure-Derivate der allgemeinen Formel I

$$\begin{array}{l} R^1 - S - N - CO - R^2 \\ \qquad\quad\;\; | \\ \qquad H - N - CO - R^2 \end{array} \qquad (I)$$

in welcher
$R^1$ eine tertiäre Alkylgruppe, nämlich tert.-Butyl oder tert.-Amyl und
$R^2$ einen Ester- oder Amidrest bedeuten.

2. 1-tert.-Butylthiohydrazin-1,2-dicarbonsäure-dimorpholid.

3. 1-tert.-Butylthiohydrazin-1,2-dicarbonsäure-di-tert.-butylester.

4. Verfahren zur Herstellung der Verbindungen gemäß Anspruch 1, dadurch gekennzeichnet, daß man ein tert.-Alkylmercaptan der allgemeinen Formel II

$$R^1—S—H \qquad (II)$$

in welcher $R^1$ die in Anspruch 1 angegebene Bedeutung besitzt, mit einem Azodicarbonsäure-Derivat der allgemeinen Formel III

$$R^2—OC—N = N—CO—R^2 \qquad (III)$$

in welcher $R^2$ die in Anspruch 1 angegebene Bedeutung besitzt, in einem inerten, aprotischen Lösungsmittel in Gegenwart einer katalytischen Menge einer sehr starken Base zur Umsetzung bringt.

5. Verfahren nach Anspruch 4, dadurch gekennzeichnet, daß man als sehr starke Base ein Alkali- oder Erdalkali-alkoholat, -amid, -hydrid oder -hydroxid einsetzt.

6. Verwendung einer Verbindung nach Anspruch 1, 2 oder 3 zur Übertragung des tert.-Butylthio-Restes oder des tert.-Amylthio-Restes an Thiole, insbesondere an Cystein, Cystein-Derivate und Cystein-Peptide.

7. Verwendung einer Verbindung nach Anspruch 1, 2 oder 3 in der Synthese von Cystin-Peptiden durch intermediären Austausch des Cystein-Thiol-Schutzes und in der recyclierbaren Subtraktion überschüssiger Cystein-Komponente in der Synthese asymmetrischer Cystin-Peptide.

## Claims

1. 1-tert.-alkyl-thiohydrazine-1,2-dicarboxylic acid derivatives of the general formula I

$$\begin{array}{l} R^1 - S - N - CO - R^2 \\ \qquad\quad\;\; | \\ \qquad H - N - CO - R^2 \end{array} \qquad (I)$$

in which
$R^1$ means a tertiary alkyl group, viz. tert.-butyl or tert.-amyl and
$R^2$ means an ester or amide radical.

2. 1-tert.-butylthiohydrazine-1,2-dicarboxylic acid dimorpholide.

3. 1-tert.-butylthiohydrazine-1,2-dicarboxylic acid-di-tert.-butyl ester.

4. A process for the preparation of the compounds according to claim 1, characterized in that a tert.-alkyl mercaptan of the general formula II

$$R^1—S—H \qquad (II)$$

in which $R^1$ has the meaning indicated in claim 1 is reacted with an azodicarboxylic derivative of the general formula III

$$R^2—OC—N = N—CO—R^2 \qquad (III)$$

in which $R^2$ has the meaning indicated in claim 1 in an inert aprotic solvent in the presence of a catalytic amount of a very strong base.

5. A process according to claim 4, characterized in that an alkali or alkaline earth alcoholate, amide, hydride or hydroxide is used, as very strong base.

6. Use of a compound according to claim 1, 2 or 3 for transferring the tert.-butylthio radical or the tert.-amylthio radical to thiols, in particular to cysteine, cysteine derivatives and cysteine peptides.

7. Use of a compound according to claim 1, 2 or 3 in the synthesis of cystine peptides by intermediary exchange of the cysteine thiol protection and in the subtraction capable of recycling of excessive cysteine component in the synthesis of asymmetric cystine peptides.

## Revendications

1. Dérivés d'acides 1-tert.-alkyl-thiohydrazine-1,2-dicarboxyliques de formule générale I

$$\begin{array}{l} R^1 - S - N - CO - R^2 \\ \qquad\quad\;\; | \\ \qquad\;\; H - N - CO - R^2 \end{array} \qquad (I)$$

dans laquelle

$R^1$ représente un groupe alkyle tertiaire, à savoir butyle tertiaire ou amyle tertiaire et

$R^2$ un radical ester ou amide.

2. Dimorpholide d'acide 1-tert.-butylthiohydrazine-1,2-dicarboxylique.

3. Ester di-tert.-butylique d'acide 1-tert.-butylthiohydrazine-1,2-dicarboxylique.

4. Procédé pour la préparation des composés selon la revendication 1, caractérisé en ce qu'on met en réaction un tert.-alkylmercaptan de formule générale II

$$R^1—S—H \qquad (II)$$

dans laquelle $R^1$ a la signification donnée dans la revendication 1, avec un dérivé d'acide azodicarboxylique de formule générale III

$$R^2—OC—N = N—CO—R^2 \qquad (III)$$

dans laquelle $R^2$ a la signification donnée dans la revendication 1, dans un solvant aprotique inerte en présence d'une quantité catalytique d'une base très forte.

5. Procédé selon la revendication 4, caractérisé en ce qu'on utilise, en tant que base très forte, un alcoolate, amidure, hydrure ou hydroxyde alcalin ou alcalinoterreux.

6. Utilisation d'un composé selon l'une quelconque des revendications 1 à 3 pour le transfert du radical tert.-butylthio ou du radical tert.-amylthio à des thiols, en particulier à la cystéine, à des dérivés de la cystéine et à des cystéine-peptides.

7. Utilisation d'un composé selon l'une quelconque des revendications 1 à 3 dans la synthèse de cystine-peptides par échange intermédiaire de la protection du thiol de la cystéine et dans la soustraction recyclable du composant cystéine en excès dans la synthèse de cystine-peptides dissymétriques.